# EUROPEAN PATENT APPLICATION

(11) **EP 0 967 287 A2**
(43) Date of publication of application: **29.12.1999**
(21) Application number: 99107615.9
(22) Date of filing: 16.04.1999
(51) Int. Cl.: C12N 15/81, C12P 25/00, C12N 15/55, C12N 9/78, A23K 1/16, A23L 1/302

(54) **Overproduction of riboflavin in yeast**

(30) Priority: 23.04.1998 EP 98107380
(71) Applicant: F. HOFFMANN-LA ROCHE AG, 4070 Basel (CH)
(72) Inventor: Babyak, Lyubov Ya., 290056 Lviv (UA); Bacher, Adelbert, 85748 Garching (DE); Boretskyy, Yuriy R., 290035 Lviv (UA); Demchyshyn, Vasyl V., 290040 Lviv (UA); Eberhardt, Sabine, 85737 Ismaning (DE); Fedorovych, Dariya, 290000 Lviv (UA); Luettgen, Holger, 85748 Garching (DE); Richter, Gerald, 85080 Gaimersheim (DE); Van Loon, Adolphus, 4310 Rheinfelden (CH)
(74) Representative: Braun, Axel

(57) **Abstract**

The present invention is directed to a yeast strain which has been transformed by a recombinant DNA sequence comprising a DNA sequence which upon expression in a suitable host cell encodes at least one polypeptide with riboflavin biosynthetic activity and which DNA sequence is transcriptionally linked to a promotor functional in such yeast strain, a process for the production of riboflavin characterized therein that such a yeast strain is cultured under suitable culture conditions and process for the production of a food or feed composition

## Description

Derivatives of riboflavin (the flavocoenzymes FMN and FAD) are universally required for redox reactions in all cellular organisms. Riboflavin (vitamin B₂) is produced by all plants and by many microorganisms [Demain A.L. Riboflavin oversynthesis. Ann. Rev. Microbiol. 1972, 26, 369]. The compound is not produced in vertebrates. Riboflavin is therefore an essential nutrient for man and animals.

Riboflavin can be produced by chemical synthesis and by various fermentation procedures using strains of *Bacillus* (e.g. *Bacillus subtilis*), the ascomycetes *Ashbya gossypii* and *Eremothecium ashbyi* [Demain A.L. Riboflavin oversynthesis. Ann. Rev. Microbiol. 1972, 26, 369 and Mitsuda H, Nakajima K., Effects of 8-azaguanine on riboflavin production and on the nucleotide pools in non-growing cells of Eremothecium ashbyii. J. Nutr Sci Vitaminol (Tokyo) 1973; 19(3):215-227], various yeast strains such as *Candida guilliermondii*, *Candida famata* [F.W. Tanner, Jr.C. Vojnovich, J.M. Van Lanen. Riboflavin production by Candida species. Nature, 1945, 101 (2616):180-181]and related strains, as well as other microorganisms.

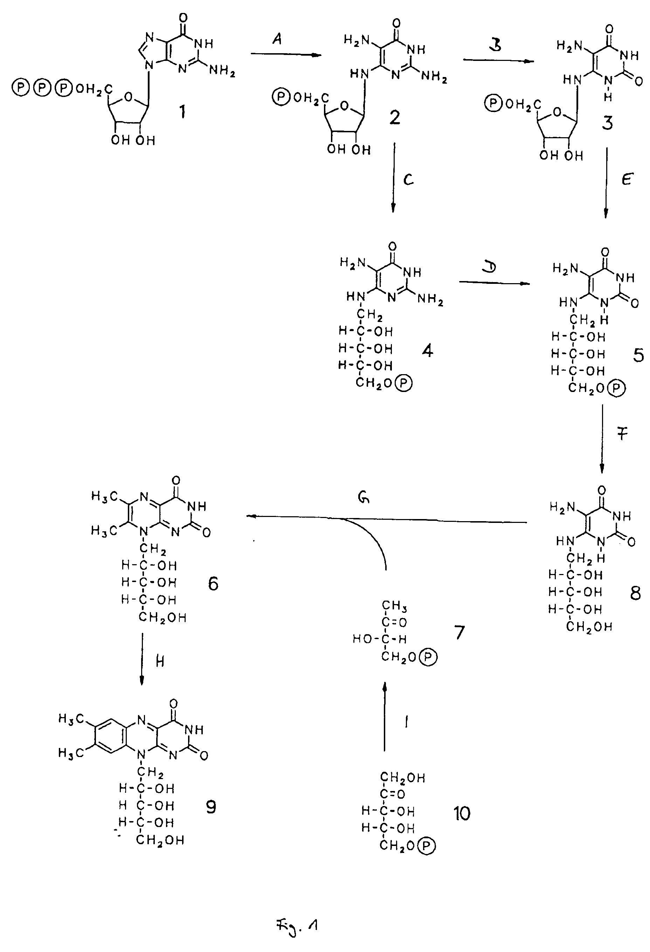

The pathway of riboflavin biosynthesis in yeast is shown in Fig. 1. The precursors for the biosynthesis of the vitamin are guanosine triphosphate (GTP) and ribulose 5-phosphate. One mol of GTP and two mol of ribulose 5-phosphate are required to biosynthetically generate one mol of riboflavin.

In the yeast, *Saccharomyces cerevisiae*, the biosynthesis of the vitamin requires at least six genes, specifically the genes *RIB1, RIB2, RIB3, RIB4, RIB5* and *RIB7* [Oltmanns O., Bacher A., Lingens F. and Zimmermann F.K. Biochemical and genetic classification of riboflavine deficient mutants of Saccharomyces cerevisiae. Mol. Gen. Genet. 1969, 105, 3061. In *C. guilliermondii,* the biosynthesis of riboflavin has also been shown to require the products of at least six genes, specifically the genes *RIB1, RIB2, RIB3, RIB4, RIB5* and *RIB6* (2). The enzymes specified by these *C. guilliermondii* genes and their roles in the biosynthetic pathway are summarized in Fig. 1. In contrast to the situation in *B. subtilis*, the riboflavin biosynthetic genes are not clustered in the eukaryots *S. cerevisiae* and *C. guilliermondii*.

The initial step in the biosynthetic pathway is the opening of the imidazole ring of GTP catalyzed by the enzyme, GTP cyclohydrolase II. The product of this enzyme has been reported to be 2,5-diamino-6-ribosylamino-4(3H)-pyrimidinone 5'-phosphate. This intermediate is converted to 5-amino-6-ribitylamino-2,4(1H,3H)-pyrimidinedione by a sequence of side chain reduction, ring deamination, and dephosphorylation. The hypothetical enzyme involved in the dephosphorylation of 5-amino-6-ribitylamino 5'-phosphate is still unknown. The conversion of 5-amino-6-ribitylamino-2,4(1H,3H)-pyrimidinedione to 6,7-dimethyl-8-ribityllumazine by the enzyme, 6,7-dimethyl-8-ribityllumazine synthase, requires a second substrate, 3,4-dihydroxy-2-butanone 4-phosphate, which is obtained from ribulose 5-phosphate by the catalytic action of 3,4-dihydroxy-2-butanone-4-phosphate synthase. Finally, 6,7-dimethyl-8-ribityllumazine is converted to riboflavin by a dismutation reaction catalyzed by riboflavin synthase. The sequence of the *RIB1* gene directing the synthesis of GTP cyclohydrolase II, the initial enzyme of the riboflavin pathway, has been established in the yeast, *C. guilliermondii* (4).

Recombinant strains of *Bacillus subtilis* for the production of riboflavin by fermentation have been described, e.g. in EP 405 370. These strains carry the riboflavin operon under the control of a strong promoter directing the production of the cognate enzymes in large amount. The gene constructs of the riboflavin operon under the control of a strong promoter can be present at one or several different locations on the *B. subtilis* chromosome. The incorporation of an additional gene of the riboflavin pathway under the control of a strong promoter at a separate locus on the *B. subtilis* chromosome has also been shown to increase the yield of riboflavin obtained by fermentation, see EP 821 063.

Whereas the production of riboflavin by strains of yeasts such as *C. guilliermondii* has been reported, recombinant DNA technology has not been applied for the overexpression of riboflavin biosynthetic genes in *C. guilliermondii* or in related flavinogenic yeasts so far.

It is therefore an object of the present invention to provide recombinant means which should allow the production of yeast strains which overproduce riboflavin. More specifically it is an object of the present invention to provide a yeast strain which has been transformed by a recombinant DNA sequence comprising a DNA sequence which upon expression in a suitable host cell encodes at least one polypeptide with riboflavin biosynthetic activity and which DNA sequence is transcriptionally linked to a promotor functional in such yeast strain and even more specifically such a yeast strain which belongs to the groups of flavinogenic yeasts which overproduce riboflavin under conditions of iron starvation like a yeast strain which is selected from the following group: Schwanniomyces, preferably Schwanniomyces occidentalis, Debaryomyces, preferably Debaryomyces kloeckeri, Torulopsis, preferably Torulopsis candida, or, especially Candida, preferably Candida guilliermondii or Candida famata (Logvinenko et al., Ukrainskii Biokhimicheskii Zhurnal 61(1), 28-32, 1989; Logvinenko et al., Mikrobiologiya 57(2), 181-186, 1988 and Nakase and Suzuki, Journal of General and Appl.Mikrobiology 31(1), 49-70 (1985). It is furthermore an object of the present invention into provide such yeast strains wherein the polypeptide encoding DNA sequence is from yeast, preferably flavinogenic yeasts which overproduce riboflavin under conditions of iron starvation, more preferably Candida, e.g. Candida guilliermondii or Candida famata.

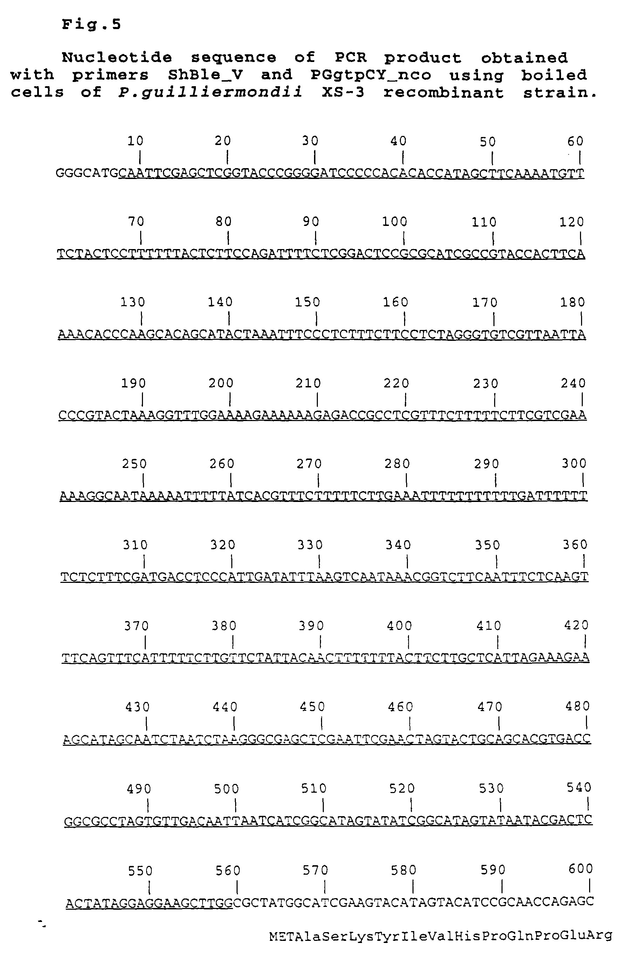

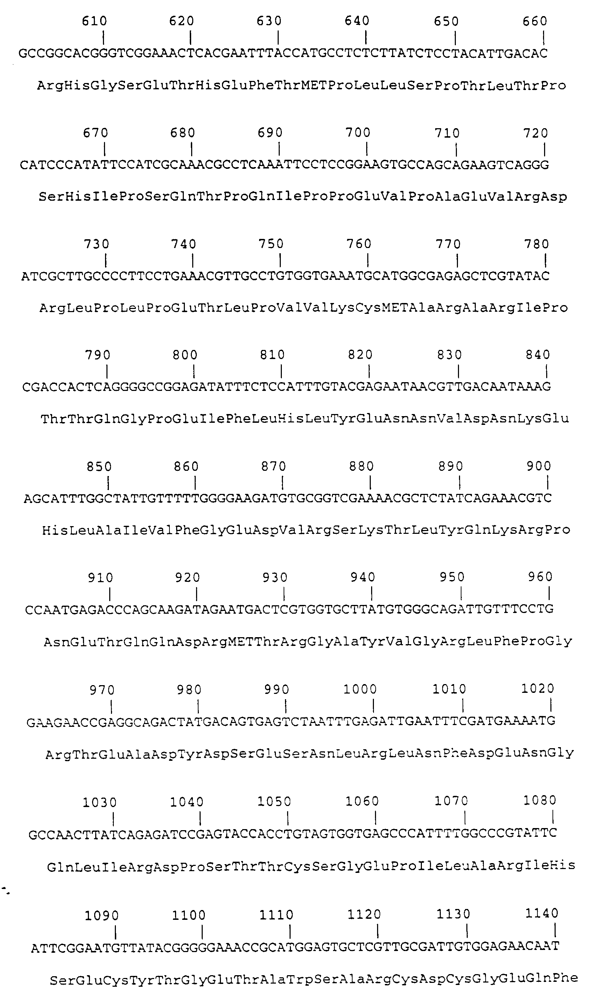

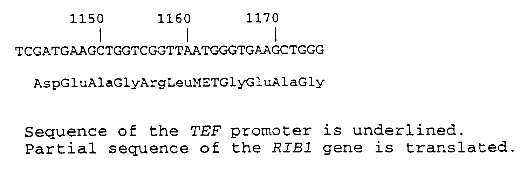

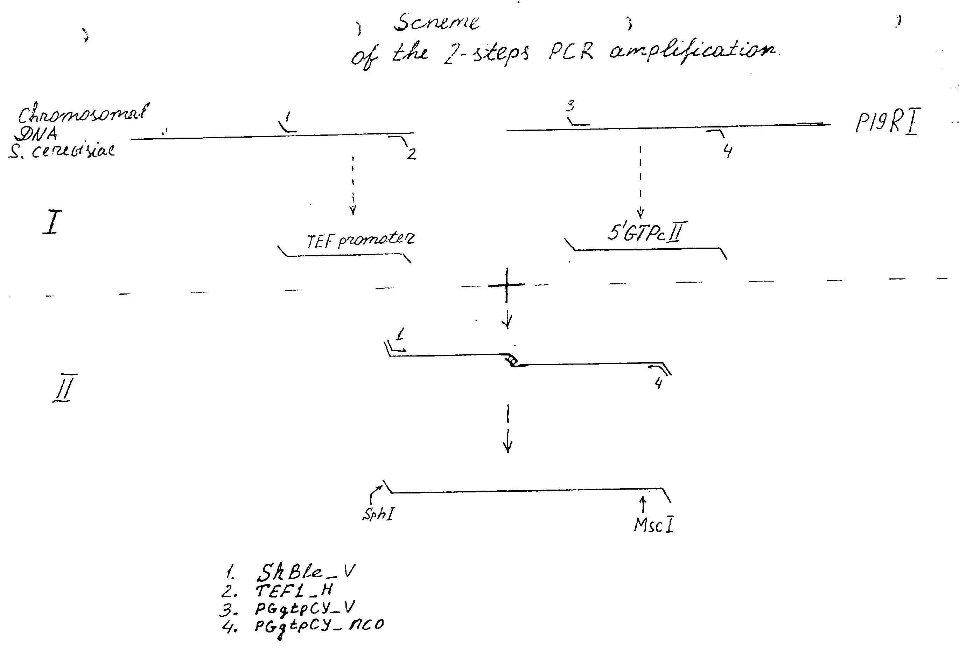

It is also an object of the present invention to provide such yeast strains wherein the polypeptide encoding DNA sequence encodes a protein with GTP cyclohydrolase II activity and is selected from the following DNA sequences:
a) the DNA sequence as shown in Fig. 5 or its complementary strand;
b) DNA sequences which hybridize under standard conditions to the protein coding regions of the DNA sequences defined in (a) or fragments thereof; and
c) DNA sequences which, but for the degeneracy of the genetic code, would hybridize to the DNA sequences defined in (a) and (b).

It is furthermore an object of the present invention to provide such yeast strains wherein the promotor is the TEF S. cerevisiae promotor.

It is also an object of the present invention to provide a process for the production of riboflavin characterized therein that a yeast strain as described above is cultured under suitable culture conditions and the riboflavin produced is isolated from the medium or the yeast strain by methods known to the man skilled in the art, and a process for the production of a food or feed composition characterized therein that riboflavin as obtained by such process is mixed with one or more suitable food or feed ingredients by a process known to the man skilled in the art.

All *C. guilliermondii* strains used in the practice of the present invention are derivatives of the *C. guilliermondii* strain obtained from the American Type Culture Collection (ATCC) under accession No. ATCC 9058 (1). Candida guilliermondii (ATCC 9058) has been redeposited as a Budapest Treaty deposit on April 1, 1998 and has been assigned accession No. ATCC 74437 *Candida guilliermondii* is a representative of yeast species which overproduce riboflavin (vitamin B2) under conditions of iron starvation.The group includes also *Schwanniomyces occidentalis,* (or called Debaryomyces occidentalis) *Debaryomyces cloeckeri*, *Torulopsis candida* and *Candida famata*. The latter species is used for industrial production of riboflavin. Regarding the taxomonic assignments of yeast species a man skilled in the art knows that these assignments are handled variably by different authors, for example: *Candida famata - Debaryomyces hansenii* - *Torulaspora hansenii*, *Candida guilliermondii* - *Pichia guilliermondii* - *Yamadazyma guilliermondii* are used as synonyms. A man skilled in the art knows that microorganisms which can be used for the practice of the present invention, either as host cells or source for the isolation of DNA sequences, are available from depository authorities, e.g. the American Type Culture Collection (ATCC), the Centraalbureau voor Schimmelcultures (CBS) or the Deutsche Sammlung fur Mikroorganismen und Zellkulturen GmbH (DSM) or any other depository authority as listed in the Journal "Industrial Property" [(1991) 1, pages 29-40].

DNA sequences useful for the practice of the present invention and encoding a polypeptide with riboflavin biosynthetic activity can be obtained from any microorganism known to produce riboflavin (see above) in form of e.g., genomic or c-DNA sequences by methods known to the man skilled in the art or by using the wellknown PCR-Technology. The principles of the polymerase chain reaction (PCR) methode are outlined e.g. by White et al., Trends in Genetics, 5, 185-189 (1989), whereas improved methods are described, e.g. in Innis et al. [PCR Protocols: A guide to Methods and Applications, Academic Press, Inc. (1990)].

The sequence information needed for the design of the PCR-primers can be obtained from any sequence data base, for example like Genbank (Intelligenetics, California, USA), European Bioinformatics Institute (Hinston Hall, Cambridge, GB), NBRF (Georgetown University, Medical Centre, Washington DC, USA) and Vecbase (University of Wisconsin, Biotechnology Centre, Madison, Wisconsin, USA).

Once such DNA sequences have been obtained they can be expressed in any desirable host and the riboflavin biosynthetic activity of the encoded polypeptide can be determined by any assay known to the man skilled in the art and described, e.g. in Bacher A., G. Richter, H. Ritz, S. Eberhardt, M.Fisher and C. Krieger, Biosynthesis of riboflavin: GTP cyclohydrolase II, deaminase, and reductase. Methods in enzymology 1997; 280: 382-389; K.Kis, R. Volk and A. Bacher, Biosynthesis of riboflavin. Studies on the reaction mechanism of 6,7-dimethyl-8-ribityllumazine synthase. Biochemistry 1995, 34, 2883-2892; Logvinenko EM, Shavlovskii GM, Zakal'skii AE, Kontorovskaia Niu. Properties of 2,5-diamino-4-oxy-6-ribosylaminopyrimidine-5 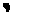 -phosphate reductase, a enzyme of the second stage of flavinogenesis in Pichia guilliermondii yeast Ukr Biokkhim Zh 1989 Jul; 61(4): 47-54; G. Richter, M. Fischer, C.Krieger, S.Eberhardt, H. Lüttgen, I. Gerstenschläger and A. Bacher. Biosynthesis of riboflavin. Characterization of the bifunctional deaminase/reducase of *Escherichia coli* and *Bacillus subtilis*. J. Bacteriol. 1997, 179, 2022-2028; K. Ritsert, D. Turk, R. Huber, R. Ladenstein, K. Schmidt-Bäse and A. Bacher. Studies on the lumazine synthase/riboflavin synthase complex of *Bacillus subtilis*. Crystal structure analysis of reconstituted icosahedral β subunit capsied at 2.4 Å resolution. J. Mol. Biol. 1995, **253**, 151-167.

The DNA sequences used for the practice of the present invention comprise at least one DNA sequence which enodes a polypeptide with riboflavin biosynthetic activity. It is however, understood by the man skilled in the art that also more then one, e.g. all enzymes of the riboflavin bipsynthetic pathway can be encoded by such DNA sequences and one or more of this enzymes can be encoded by DNA sequences of different species origin or can be of partial or total synthetic origin as long as they show at least one desired riboflavin biosynthetic activity. One example of such a DNA sequence is given in Fig. 5 coding for a GTP cyclohydrolase II. However, DNA sequences which hybridize under standard conditions to this DNA sequence and encode such a GTP cyclohydrolase are also useful for the practice of the present invention.

"Standard conditions" for hybridization in this context are conditions which are generally used by a man skilled in the art to detect specific hybridization signals and which are described, e.g. by Sambrook et al., "Molecular Cloning" second edition, Cold Spring Harbor Laboratory Press 1989, New York, or preferably so called stringent hybridization and non-stringent washing conditions or more preferably so called stringent hybridization and stringent washing conditions a man skilled in the art is familiar with and which are described, e.g. in Sambrook et al. (s.a.). "Fragment of the DNA sequences" means in this context a fragment which codes for a polypeptide still having the enzymatic activity as specified above.

For the overexpression of the proteins encoded by the DNA sequences of the present invention these sequences can be linked to promoters which are functional in the desired yeast and are, e.g. the S.cerevisiae TEF-promotor (see Example 2) or the pho5-promotor [Vogel et al., Mol. Cell. Biol., 2050-2057 (1989); Rudolf and Hinnen, Proc, Natl. Acad. Sci. 84, 1340-1344 (1987)] or the gap-promotor or the aox1-promotor [Koutz et al., Yeast 5, 167-177 (1989; Sreekrishna et al., J. Basic Microbiol. 28, 265-278 (1988)] or the FMD promoter [Hollenberg et al., EP 299108] or MOS-promotor [Ledeboer et al., Nucleic Acids Res. 13, 3063-3082 (1985)].

The DNA sequences useful for the practice of the present invention can also comprise so called "ARS" elements (autonomounsly replicating sequence) as described, e.g. in Example 1.

### Examples

If not specifically indicated or referred to by references standard procedures have been used as described, e.g. in Sambrook et al. "Molecular Cloning", (s.a.) and Cregg, J.M., K.J. Barriner, A.Y.Hessler, and K.R. Madden (1985). Pichia pastoris as a host system for transformations. Mol. Cell. Biol. 5, 3376-3385.

### Example 1

### Autonomous replication of plasmid p19R1 in C. guilliermondii

The plasmid pFR1 carrying the RIB1 gene of *C. guilliermondii* has been described (Zakalsky at al. *Genetika* 26, 614-620, 1990).In order to subclone the RIB1 gene, plasmid pFR1 was digested with the restriction nuclease *Sal*I. The resulting fragments were cloned into the *Sal*I site of the pUC19 vector. The ligation mixture was transformed into the *E. coli* mutant strain BSV821 carrying a mutation of the *ribA* gene conducing to riboflavin deficiency. Colonies growing in the absence of riboflavin were isolated and were shown to contain a plasmid p19R1.

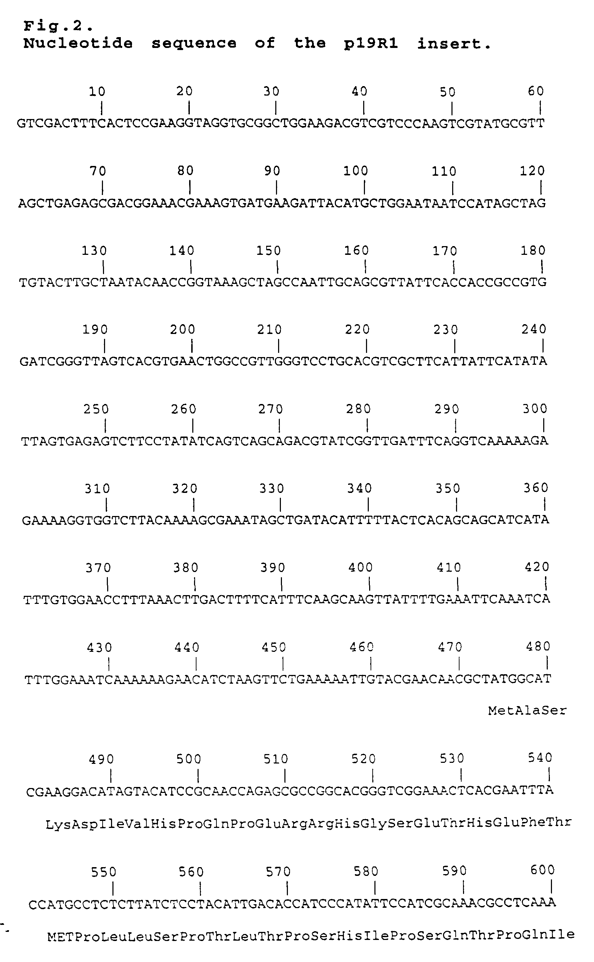

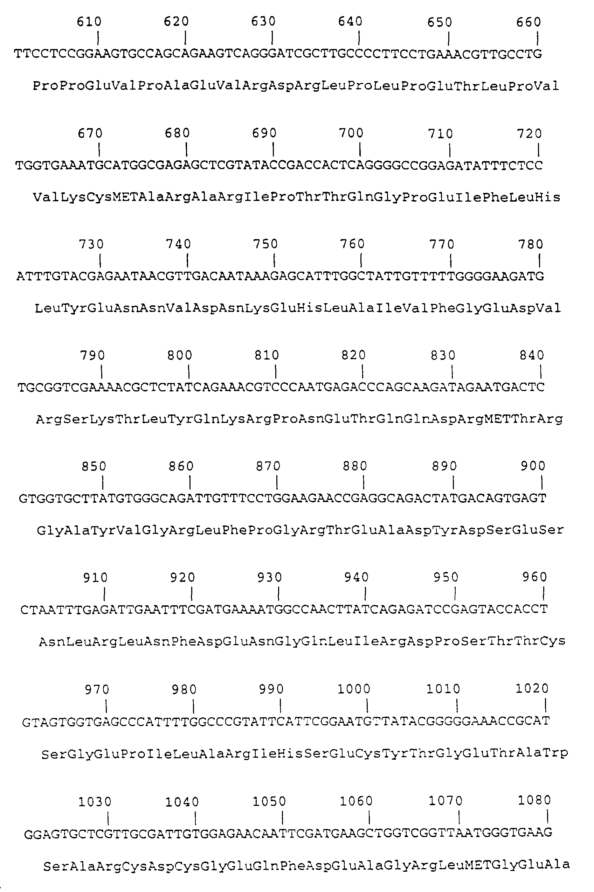

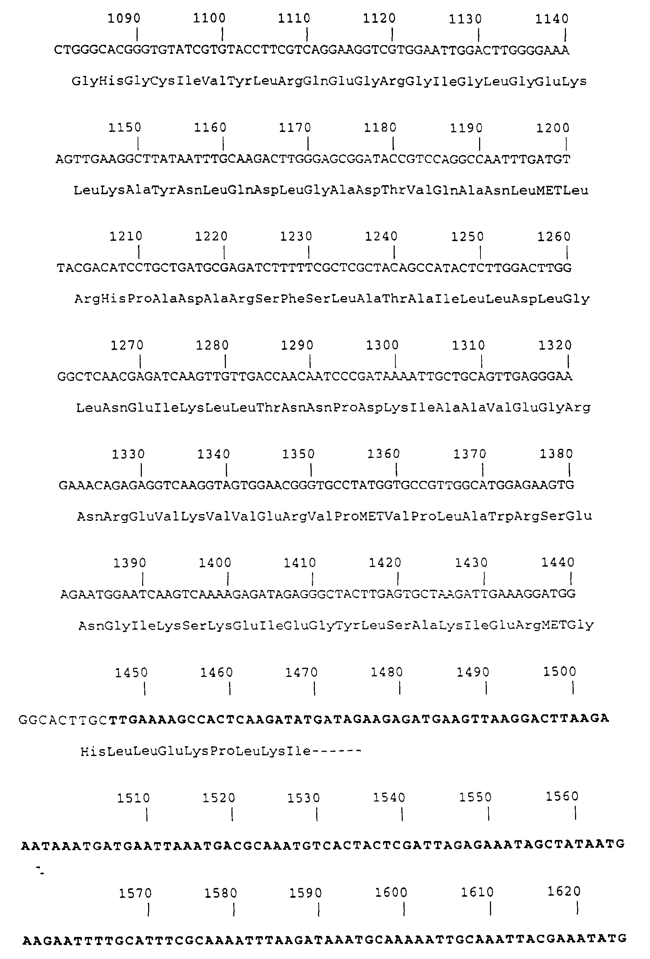

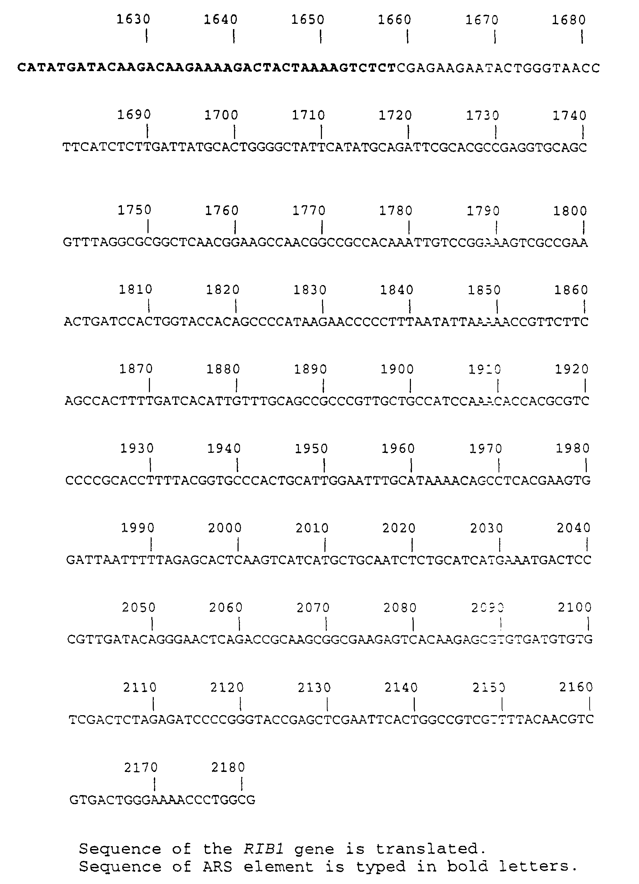

The plasmid p19R1 was sequenced and was shown to contain a 2.18 kb fragment of *C. guilliermondii* DNA in the *Sal*I site of the pUC19 vector. The sequence of this insert is shown in Fig. 2.

The DNA sequence shown in Fig. 2 carries the *RIB1* gene of *C*. *guilliermondii.* The plasmid transforms *C. guilliermondii* mutant defective in the *RIB1* gene to riboflavin prototrophy and can replicate autonomously in this yeast species. The replication was shown to be due to the presence of an autonomously replicating sequence (ARS) comprising approximately base pairs 1542 to 1755 in Fig. 2 and extending into the structural gene *RIB1*.

### Example 2

### Construction of a plasmid for hyperexpression of the RIB1 gene of C. guilliermondii

The *TEF* gene of the yeast, *S. cerevisiae*, specifies the translation elongation factor 1-alpha. This gene is known to be transcribed in *S. cerevisiae* with high efficiency.

A DNA fragment carrying the *S. cerevisiae TEF* promoter and the 5' part of the the *RIB1* gene of *C.guilliermondii* was obtained by PCR amplification. Initially, a DNA sequence located upstream from the 5' end of the *S. cerevisiae TEF* gene was amplified by PCR with primers ShBle_V and TEF1_H using chromosomal DNA of *S. cerevisiae* as a template. The amplified DNA fragment (subsequently designeted *TEF* promoter) comprises bp 15 984 to 16 344 of the sequence listed under EMBL accession number gb/U51033/YSCP9513.

Independently, a DNA fragment comprising the 5'-terminal part of *C. guilliermondii* GTP cyclohydrolase II structural gene was obtained by PCR with primers PGgtpCY_V and PGgtpCY_nco using p19R1 plasmid as a template. The amplified DNA fragment (subsequently designeted 5'GTPcII) comprises bp 460 to 1145 of the sequence shown in Fig. 2.

The DNA amplificates obtained in the two PCR reactions described above comprising parts of the *TEF* gene of *S. cerevisiae* and of the 5'GTPcII were mixed, and a third PCR amplification was performed using the primers ShBle_V and PGgtpCY_nco. This reaction yielded a DNA fragment which contains the complete *TEF* promoter and the 5' part of the *C.guilliermondii RIB1* gene.

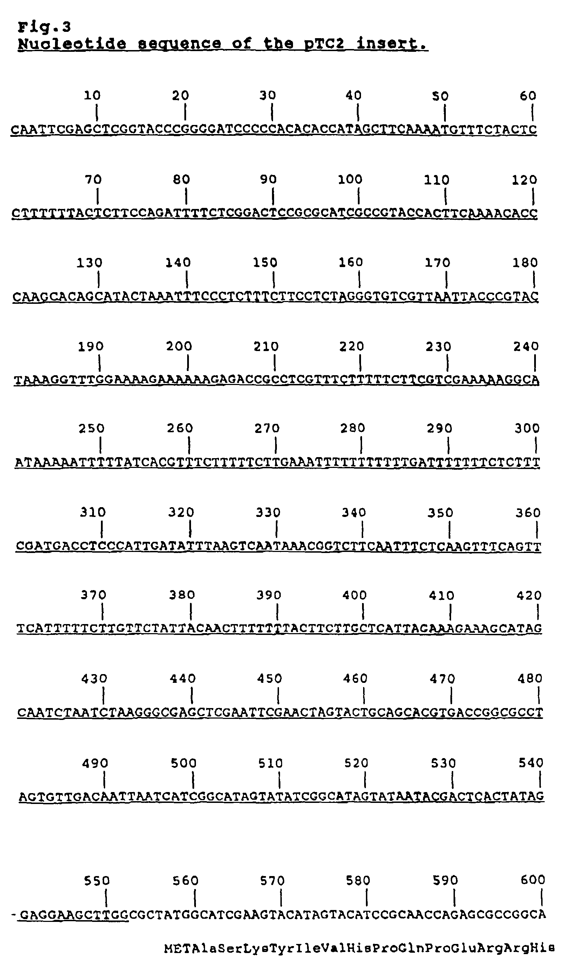

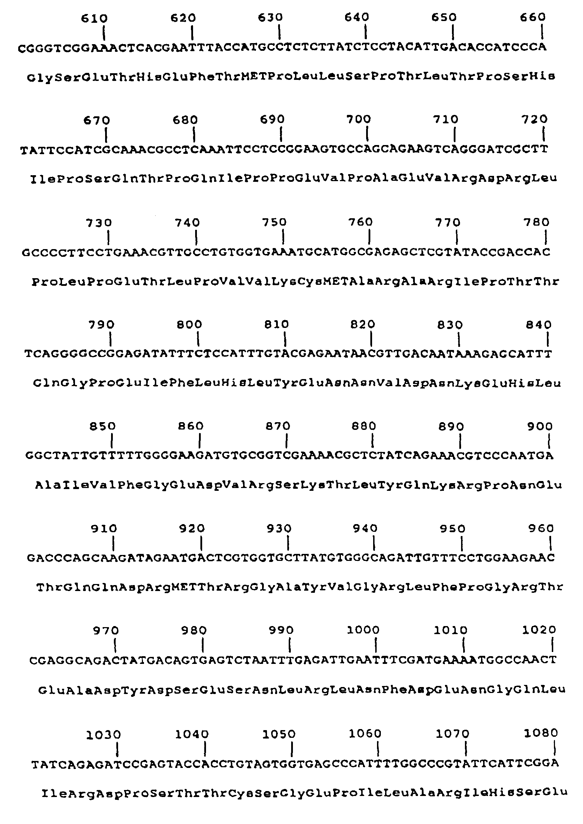

All primers used in these PCRs are shown in Table 2. The sequence of the final amplificate is shown in Fig. 3.

The amplificate contains a cutting site for the restriction nucleases *Sph*I which had been introduced via the primer ShBle_V. The amplificate also contains an MscII site which is a feature of the RIB1 gene. The amplificate was digested with *Sph*I and *Msc*I.

The plasmid p19R1 (whose constructions is described above) was also digested with the same enzymes, and the PCR amplificate was ligated into the digested plasmid.

The ligation mixture was transformed into a mutant designated Rib7 of *Escherichia coli* carrying a mutation of the *ribA* gene which specifies GTP cyclohydrolase II. Transformation was performed by electroporation according to the protocol of Invitrogene (5).

The *E. coli* cells were plated on Luria-Broth plates supplemented with ampicillin (100 mg/ml) which did not contain riboflavin. Colonies growing on this medium were isolated and were shown to contain a plasmid designated pTC2.

The sequence of the insert of the plasmid pTC2 is shown in Fig. 3.

Plasmid pTC2 was digested with *Xho*I and *Sal*I restriction endonucleases yielding 4.4 kb and 0.5 kb fragments. The 4.4 kb fragment was circularized with T4 DNA ligase. The ligation mixture was transformed into the Rib7 mutant of *E. coli* carrying the RibA mutation. Transformation was achieved by electroporation. The cells were plated on Luria-Broth plates containing ampicillin and no riboflavin (see above). Colonies growing on these plates were isolated and were shown to contain a plasmid pTCdXS2. The procedure had resulted in the removal of 0.5 kb base pairs from the plasmid pTC2.

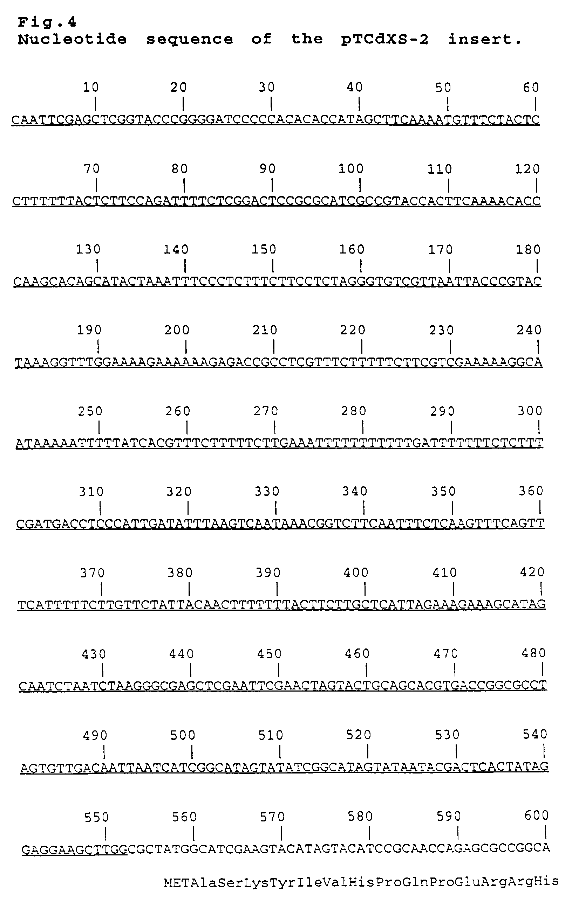

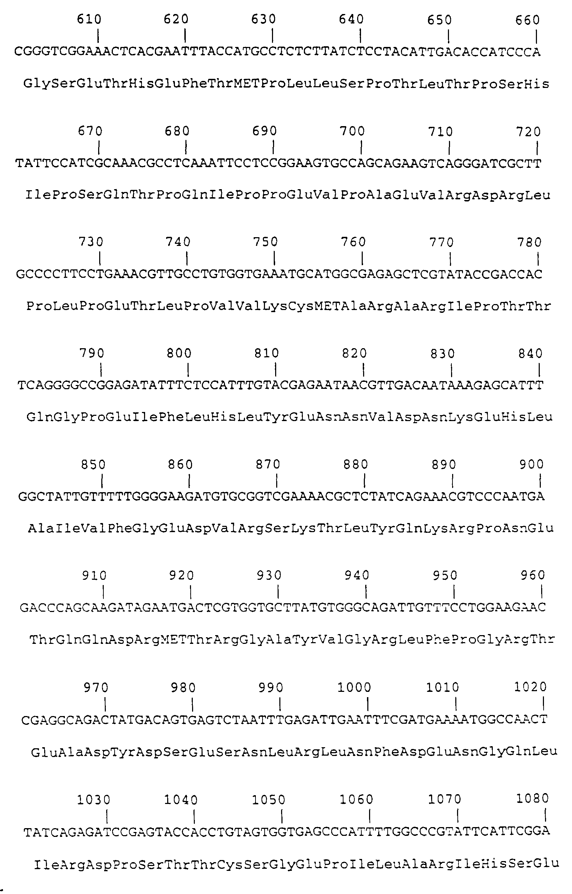

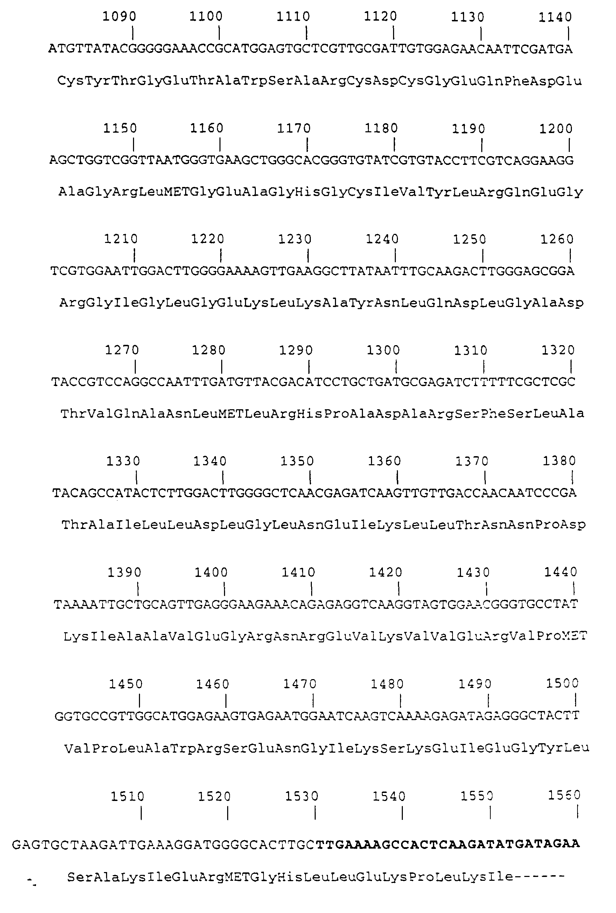

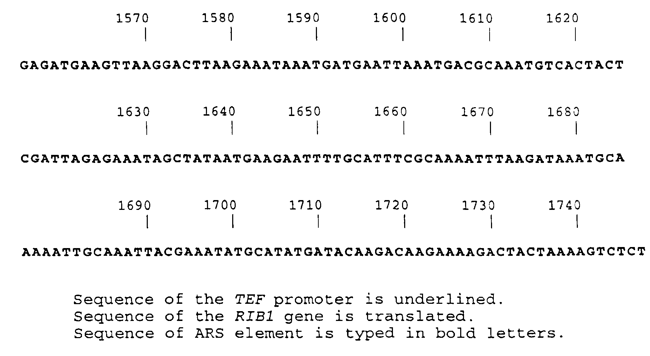

The sequence of the insert of the plasmid pTCdXS2 is shown in Fig. 4. The open reading frame of the *RIB1* gene of *C.guilliermondii* is indicated. The DNA segment representing the promoter of the *TEF* gene of *S. cerevisiae* is also shown.

### Example 3

### Construction of a recombinant C. guilliermondii strains

The riboflavin deficient mutant rh-21 with an apparent defect of the *RIB1* gene specifying GTP cyclohydrolase II (2) has been obtained after chemical mutagenesis of the L2 strain (2) which was previously obtained from the ATCC 9058 *C. guilliermondii* strain.

The plasmid pTCdXS2 was transformed into the *RIB1* mutant strain rh-21 of *C. guilliermondii* by the LiCl procedure, respectively (6). The cells were plated on YPD medium without added riboflavin. Colonies growing without riboflavin were isolated. They were monitored for GTP cyclohydrolase II activity and for riboflavin production as described below.

The prototrophic strains were monitored for the presence of DNA segments introduced with the plasmid by PCR analysis. PCR was performed using the primers ShBle_V and PGgtpCY_nco and boiled *C. guilliermondii* recombinant strains cells as template. Primer ShBle_V is complementary to the *TEF* promoter and primer PGgtpCY_nco is complementary to the *RIB1* structural gene. Amplificates of the expected length (1175 base pairs) were obtained from all transformants isolated. The amplificate obtained from strain XS-3 was isolated and was sequenced by the fluorescent dideoxy terminator method. The sequence is shown in Fig. 5. This sequence is identical with base pairs 1 to 1168 of the insert of plasmid pTCdXS2.

The recombinant transformants were genetically stable. Specifically, they did not segregate riboflavin deficient subclones.

### Example 4

### GTP cyclohydrolase activity in recombinant C. guilliermondii strains

The level of GTP cyclohydrolase II activity in the recombinant strains described above was determined as follows.

The recombinant *C. guilliermondii* cells were grown aerobically in synthetic Burkholder medium supplemented with trace elements (Science 101, 180, 1945) but without asparagine, during 2-3 days at 30°C. *C. guilliermondii* L2 strain (wild type) served as a control in these experiments.

Cells from exponential growth phase were harvested by centrifugation (5000 g, 15 min), washed twice with 20 mM Tris HCl, pH 8.2, containing 1 mM DTT and 1 mM MgCl₂. Cells were stored at -20°C. Frozen cells mass (1-3 g) was thawed in 3-9 ml of washing buffer. Cells were disrupted by agitation with glass beads (d = 0.8 mm). After centrifugation, cell extract was dialyzed overnight against 100 volumes of washing buffer. Protein concentration was measured by the Lowry method.

Reaction mixtures for GTP cyclohydrolase assays contained 20 mM Tris HCl, pH 8.2, 3 mM DTT, 2 mM MgCl₂, 1 mM GTP, and protein (protein concentration, 1-3 mg/ml, total volume, 4 ml). They were incubated at 37°C for 20 min in the dark.

After incubation, 2 ml aliquots were removed, and 2,3-butanedione was added to a final concentration of 0.5 mg/ml. The mixtures were incubated at 95°C for 30 min. Blank values were processed in the same way but without added diacetyl.

Differences in specific fluorescence of both types of aliquots were determined and were used to calculate the concentrations of 6,7-dimethylpteridin and the activity of GTP cyclohydrolase II. Results are shown in Table 3.

Strain L2 from which the mutant rh-21 had been derived was used as a control. The enzyme activity in strain L2 was 2,9 nmol mg⁻¹ h⁻¹. No enzyme activity was found in the riboflavin deficient recipient strain rh-21 carrying a mutation of the *RIB1* gene. The recombinant strains obtained by transformation with plasmid pTCdXS2 showed enzyme levels between 6,5 and 13,4 nmol mg⁻¹ h⁻¹ Thus, the enzyme level in recombinant strains was 2.3 - 4.6-fold higher as compared with the *C. guilliermondii* strain L2.

The activity of riboflavin synthase was also measured in the recombinant strains. The activity of riboflavin synthase was not affected by the transformation with the plasmids p19RI, pTC2, pTCdXS2. All strains analyzed had riboflavin synthase activities in the range of 20 nmol mg⁻¹ h⁻¹ (Table 3).

### Example 5

### Production of riboflavin by recombinant C. guilliermondii strains

*C. guilliermondii* strains (wild type and recombinant strains) were grown aerobically in synthetic Burkholder medium supplemented with trace elements [Science 101, p. 180, (1945)] but without asparagine during 4 days at 30 °C [F.W. Tanner, Jr.C. Vojnovich, J.M. Van Lanen, Riboflavin production by Candida species. Nature, 1945, 101 (2616): 180-181]. The suspension was centrifuged. Riboflavin concentration was determined fluorometrically. Results are shown in Table 4.

The wild strain L2 produced 1.2 mg riboflavin per liter under the conditions described. The recombinant strain XS-3 produced a 3-fold increased level of riboflavin (3.6 mg/l).

### Example 6

### Isolation of riboflavin

Two Erlenmeyer flasks (2.5 l) each containing 0.5 l of synthetic Burkholder medium containing trace elements but no asparagine were inoculated with the recombinant *C. guilliermondii* strain XS-3. The cultures were incubated with shaking at 30°C for 50 h. The solution was centrifuged. The supernatant was passed through a column of Florisil (4 ml bed volume) at a velocity of 500 ml/h. The column was washed with distilled water (7 ml). Riboflavin was eluted by a mixture of acetone/1M aqueous NH₄OH. The effluent was evaporated to dryness. The yield of riboflavin was determined photometrically.

### References

1. Sibirny, A. A., Zharova, V. P., Kshanovskaya, B. V., Shavlovsky, G. M. Selection of a genetic line of yeast *Pichia guilliermondii* capable to formation of significant amount of spores. *Tsytologia i genetika* 11, 330-333, 1977 (in Russian).
2. Shavlovskyy, G. M., Sibirnyy, A. A., Kshanovs'ka, B. V. Genetical classification of *C. guilliermondii* riboflavin auxotroph mutants. *Genetika* 15, 1561-1568, 1979 (in Russian).
3. Zakalsky, A. E., Zlochevsky, M. L., Stasiv, Y. Z., Logvynenko, E. M., Beburov, M. Y., Shavlovsky, G. M. Cloning of the *Pichia guilliermondii RIB1* gene coding for GTP cyclohydrolase II - first enzyme of flavinogenesis - in *E. coli* cells. *Genetika* 26, 614-620, 1990 (in Russian).
4. Liauta-Teglivets, O., Hasslacher, M., Boretskyy, Y., Kohlwein, S. D., Shavlovskii, G. M. Molecular cloning of the GTP cyclohydrolase. Structural gene *RIB1* of *Pichia guilliermondii* involved in riboflavin biosynthesis. *Yeast* 11, 945-952, 1995.
5. Zero Background/Kan Cloning Kit. Version A. 151204. Instruction manual. INVITROGENE.
6. Logvinenko, E. M., Stasiv, Yu. Z., Zlochevsky, M. L., Voronovsky, A. Ya., Beburov, M. Yu., Shavlovsky, G. M. Cloning of the *RIB7* gene encoding the riboflavin synthase of the yeast *Pichia guilliermondii. Genetika* 29, 922-927, 1993 (in Russian).

**Table 1**

| Enzymes and genes of the riboflavin pathway | | | | |
|---|---|---|---|---|
| | Enzyme | Gene | | |
| | | *S. cerevisiae* | *C. guilliermondii* | *E. coli* |
| A | GTP cyclohydrolase | *RIB1* | *RIB1* | *ribA* |
| B | bacterial deaminase | | | *ribD* |
| C | yeast reductase | *RIB7* | *RIB2* | |
| D | yeast deaminase | *RIB2* | *RIB3* | |
| E | bacterial reductase | | | *ribD* |
| F | unknown phosphatase | | | |
| G | lumazine synthase | *RIB4* | *RIB5* | *ribE* |
| H | riboflavin synthase | *RIB5* | *RIB7* | *ribC* |
| I | 3,4-dihydroxy-2-butanon 4-phosphate synthase | *RIB3* | *RIB6* | *ribB* |

**Table 2**

| Nucleotide sequences of the primers used. | | |
|---|---|---|
| **N** | **Primer** | **Sequence (5' - 3')** |
| 1 | ShBle_V | GGGCATGCAATTCGAGCTCGGTACCCG |
| 2 | TEB1_H | CGACTCACTATAGGAGGAAGCTTGGCGC |
| 3 | PGgtpCY_V | AGGAGGAAGCTTGGCGCTATGGCATCGAAGG |
| 4 | PGgtpCY_nco | GCTGGTCGGTTAATGGGTGAAGCTGGG |

**Table 3**

| Activity of GTP cyclohydrolase II and riboflavin synthase in *C. guilliermondii* recombinant strains (time of growth: 40-48 h). | | | | |
|---|---|---|---|---|
| N | Strain | Riboflavin synthase activity nmol mg⁻¹ h⁻¹ | GTP cyclohydrolase II activity nmol mg⁻¹ h⁻¹ | Ratio* |
| 1 | L2 (wild type) | 21.6 | 2.88 | 1.00 |
| 2 | R1-1 | n.d. | 10.08 | 3.50 |
| 3 | R1-2 | n.d. | 4.20 | 1.46 |
| 4 | R1-3 | 20.4 | 8.76 | 3.04 |
| 5 | R1-4 | 19.8 | 7.80 | 2.70 |
| 6 | R1-5 | 21.6 | 7.80 | 2.70 |
| 7 | TC-1 | 20.4 | 9.60 | 3.33 |
| 8 | TC-2 | n.d. | 8.40 | 2.92 |
| 9 | TC-3 | n.d. | 7.56 | 2.63 |
| 10 | XS-1 | 22.8 | 13.38 | 4.60 |
| 11 | XS-2 | n.d. | 12.60 | 4.37 |
| 12 | XS-3 | n.d. | 6.60 | 2.29 |

| | | | | |
|---|---|---|---|---|
| n.d. not determined. | | | | |
| * GTP cyclohydrolase activity of recombinant strain divided by GTP cyclohydrolase activity of strain L2 | | | | |

**Table 4**

| Riboflavin production by recombinant *C. guilliermondii* strains (time of growth: 110 h. incubation temperature: 30 °C). | | | |
|---|---|---|---|
| N | Strain | Riboflavin production [mg/l] | Relative riboflavin production |
| 1 | L2 (wild type) | 1.2 | 1.0 |
| 4 | R1-3 | 1.4 | 1.2 |
| 5 | R1-4 | 3.6 | 3.0 |
| 6 | R1-5 | 3.0 | 2.5 |
| 7 | TC-1 | 1.4 | 1.2 |
| 9 | TC-3 | 1.3 | 1.0 |
| 12 | XS-3 | 3.6 | 3.0 |
| 13 | XS-4 | 2.0 | 1.7 |
| 14 | XS-5 | 2.3 | 1.9 |

## Claims

1. A yeast strain which has been transformed by a recombinant DNA sequence comprising a DNA sequence which upon expression in a suitable host cell encodes at least one polypeptide with riboflavin biosynthetic activity and which DNA sequence is transcriptionally linked to a promotor functional in such yeast strain.

2. A yeast strain of claim 1 which belongs to the group of flavinogenic yeasts which overproduce riboflavin under conditions of iron starvation.

3. A yeast strain of claim 2 which is selected from the following group: Schwanniomyces, preferably Schwanniomyces occidentalis, Debaryomyces, preferably Debaryomyces kloeckeri, Torulopsis, preferably Torulopsis candida, or Candida, preferably Candida guilliermondii or Candida famata.

4. The yeast strain of claim 3 which is Candida guilliermondii or Candida famata.

5. A yeast strain as claimed any one of claims 1 to 4 wherein the polypeptide encoding DNA sequence is from yeast, preferably flavinogenic yeasts which overproduce riboflavin under conditions of iron starvation more preferably Candida, e.g. Candida guilliermondii or Candida famata.

6. A yeast strain as claimed in any one of claims 1 to 4, wherein the polypeptide encoding DNA sequence encodes a protein with GTP cyclohydrolase II activity and is selected from the following DNA sequences:
a) the DNA sequence as shown in Fig. 5 or its complementary strand;
b) DNA sequences which hybridize under standard conditions to the protein coding regions of the DNA sequences defined in (a) or fragments thereof; and
c) DNA sequences which, but for the degeneracy of the genetic code, would hybridize to the DNA sequences defined in (a) and (b).

7. A yeast strain as claimed in any one of claims 1 to 6 wherein the promotor is the TEF S. cerevisiae promotor.

8. A process for the production of riboflavin characterized therein that a yeast strain as claimed in any one of claims 1 to 7 is cultured under suitable culture conditions and the riboflavin produced is isolated from the medium or the yeast strain by methods known to the man skilled in the art.

9. A process for the production of a food or feed composition characterized therein that riboflavin as obtained by the process of claim 8 is mixed with one or more suitable food or feed ingredients.


SEQUENCE LISTING

<110> F. Hoffmann-La Roche AG

<120> Overproduction of riboflavin in yeast

<130> Case 20071 EP1

<140> EP-A 99.107615.8
<141> 1999-04-16

<150> 98.107380.2
<151> 1998-04-23

<160> 12

<170> PADAT Sequenzmodul, Version 1.0

<210> 1
<211> 27
<212> DNA
<213> artificial sequence

<220>
<223> primer

<400> 1

gggcatgcaa ttcgagctcg gtacccg                                          27

<210> 2
<211> 28
<212> DNA
<213> artificial sequence

<220>
<223> primer

<400> 2

cgactcacta taggaggaag cttggcgc                                         28

<210> 3
<211> 31
<212> DNA
<213> artificial sequence

<220>
<223> Primer

<400> 3

aggaggaagc ttggcgctat ggcatcgaag g                                     31

<210> 4
<211> 31
<212> DNA
<213> artificial sequence

<220>
<223> Primer

<400> 4

aggaggaagc ttggcgctat ggcatcgaag g                                     31

<210> 5
<211> 2181
<212> DNA
<213> Candida guilliermondii

<220>
<223> RIB 1 Gene

<400> 5

gtcgactttc actccgaagg taggtgcggc tggaagacgt cgtcccaagt cgtatgcgtt 60

agctgagagc gacggaaacg aaagtgatga agattacatg ctggaataat ccatagctag 120

tgtacttgct aatacaaccg gtaaagctag ccaattgcag cgttattcac caccgccgtg 180

gatcgggtta gtcacgtgaa ctggccgttg ggtcctgcac gtcgcttcat tattcatata 240

ttagtgagag tcttcctata tcagtcagca gacgtatcgg ttgatttcag gtcaaaaaga 300

gaaaaggtgg tcttacaaaa gcgaaatagc tgatacattt ttactcacag cagcatcata 360

tttgtggaac ctttaaactt gacttttcat ttcaagcaag ttattttgaa attcaaatca 420

tttggaaatc aaaaaagaac atctaagttc tgaaaaattg tacgaacaac gctatggcat 480

cgaaggacat agtacatccg caaccagagc gccggcacgg gtcggaaact cacgaattta 540

ccatgcctct cttatctcct acattgacac catcccatat tccatcgcaa acgcctcaaa 600

ttcctccgga agtgccagca gaagtcaggg atcgcttgcc ccttcctgaa acgttgcctg 660

tggtgaaatg catggcgaga gctcgtatac cgaccactca ggggccggag atatttctcc 720

atttgtacga gaataacgtt gacaataaag agcatttggc tattgttttt ggggaagatg 780

tgcggtcgaa aacgctctat cagaaacgtc ccaatgagac ccagcaagat agaatgactc 840

gtggtgctta tgtgggcaga ttgtttcctg gaagaaccga ggcagactat gacagtgagt 900

ctaatttgag attgaatttc gatgaaaatg gccaacttat cagagatccg agtaccacct 960

gtagtggtga gcccattttg gcccgtattc attcggaatg ttatacgggg gaaaccgcat 1020

ggagtgctcg ttgcgattgt ggagaacaat tcgatgaagc tggtcggtta atgggtgaag 1080

ctgggcacgg gtgtatcgtg taccttcgtc aggaaggtcg tggaattgga cttggggaaa 1140

agttgaaggc ttataatttg caagacttgg gagcggatac cgtccaggcc aatttgatgt 1200

tacgacatcc tgctgatgcg agatcttttt cgctcgctac agccatactc ttggacttgg 1260

ggctcaacga gatcaagttg ttgaccaaca atcccgataa aattgctgca gttgagggaa 1320

gaaacagaga ggtcaaggta gtggaacggg tgcctatggt gccgttggca tggagaagtg 1380

agaatggaat caagtcaaaa gagatagagg gctacttgag tgctaagatt gaaaggatgg 1440

ggcacttgct tgaaaagcca ctcaagatat gatagaagag atgaagttaa ggacttaaga 1500

aataaatgat gaattaaatg acgcaaatgt cactactcga ttagagaaat agctataatg 1560

aagaattttg catttcgcaa aatttaagat aaatgcaaaa attgcaaatt acgaaatatg 1620

catatgatac aagacaagaa aagactacta aaagtctctc gagaagaata ctgggtaacc 1680

ttcatctctt gattatgcac tggggctatt catatgcaga ttcgcacgcc gaggtgcagc 1740

gtttaggcgc ggctcaacgg aagccaacgg ccgccacaaa ttgtccggaa agtcgccgaa 1800

actgatccac tggtaccaca gccccataag aacccccttt aatattaaaa accgttcttc 1860

agccactttt gatcacattg tttgcagccg cccgttgctg ccatccaaac accacgcgtc 1920

ccccgcacct tttacggtgc ccactgcatt ggaatttgca taaaacagcc tcacgaagtg 1980

gattaatttt tagagcactc aagtcatcat gctgcaatct ctgcatcatg aaatgactcc 2040

cgttgataca gggaactcag accgcaagcg gcgaagagtc acaagagcgt gtgatgtgtg 2100

tcgactctag agatccccgg gtaccgagct cgaattcact ggccgtcgtt ttacaacgtc 2160

gtgactggga aaaccctggc g 2181

<210> 6
<211> 332
<212> PRT
<213> Candida guilliermondii

<400> 6

Met Ala Ser Lys Asp Ile Val His Pro Gln Pro Glu Arg Arg His Gly
1               5                   10                  15
Ser Glu Thr His Glu Phe Thr Met Pro Leu Leu Ser Pro Thr Leu Thr
            20                  25                  30
Pro Ser His Ile Pro Ser Gln Thr Pro Gln Ile Pro Pro Glu Val Pro
        35                  40                  45
Ala Glu Val Arg Asp Arg Leu Pro Leu Pro Glu Thr Leu Pro Val Val
    50                  55                  60
Lys Cys Met Ala Arg Ala Arg Ile Pro Thr Thr Gln Gly Pro Glu Ile
65                  70                  75                  80
Phe Leu His Leu Tyr Glu Asn Asn Val Asp Asn Lys Glu His Leu Ala
                85                  90                  95
Ile Val Phe Gly Glu Asp Val Arg Ser Lys Thr Leu Tyr Gln Lys Arg
            100                 105                 110
Pro Asn Glu Thr Gln Gln Asp Arg Met Thr Arg Gly Ala Tyr Val Gly
        115                 120                 125
Arg Leu Phe Pro Gly Arg Thr Glu Ala Asp Tyr Asp Ser Glu Ser Asn
    130                 135                 140
Leu Arg Leu Asn Phe Asp Glu Asn Gly Gln Leu Ile Arg Asp Pro Ser
145                 150                 155                 160
Thr Thr Cys Ser Gly Glu Pro Ile Leu Ala Arg Ile His Ser Glu Cys
                165                 170                 175
Tyr Thr Gly Glu Thr Ala Trp Ser Ala Arg Cys Asp Cys Gly Glu Gln
            180                 185                 190
Phe Asp Glu Ala Gly Arg Leu Met Gly Glu Ala Gly His Gly Cys Ile
        195                 200                 205
Val Tyr Leu Arg Gln Glu Gly Arg Gly Ile Gly Leu Gly Glu Lys Leu
    210                 215                 220
Lys Ala Tyr Asn Leu Gln Asp Leu Gly Ala Asp Thr Val Gln Ala Asn
225                 230                 235                 240
Leu Met Leu Arg His Pro Ala Asp Ala Arg Ser Phe Ser Leu Ala Thr
                245                 250                 255
Ala Ile Leu Leu Asp Leu Gly Leu Asn Glu Ile Lys Leu Leu Thr Asn
            260                 265                 270
Asn Pro Asp Lys Ile Ala Ala Val Glu Gly Arg Asn Arg Glu Val Lys
        275                 280                 285
Val Val Glu Arg Val Pro Met Val Pro Leu Ala Trp Arg Ser Glu Asn
    290                 295                 300
Gly Ile Lys Ser Lys Glu Ile Glu Gly Tyr Leu Ser Ala Lys Ile Glu
305                 310                 315                 320
Arg Met Gly His Leu Leu Glu Lys Pro Leu Lys Ile
                325                 330

<210> 7
<211> 1080
<212> DNA
<213> unknown

<220>
<223> final amplificate

<400> 7

caattcgagc tcggtacccg gggatccccc acacaccata gcttcaaaat gtttctactc 60

cttttttact cttccagatt ttctcggact ccgcgcatcg ccgtaccact tcaaaacacc 120

caagcacagc atactaaatt tccctctttc ttcctctagg gtgtcgttaa ttacccgtac 180

taaaggtttg gaaaagaaaa aagagaccgc ctcgtttctt tttcttcgtc gaaaaaggca 240

ataaaaattt ttatcacgtt tctttttctt gaaatttttt tttttgattt ttttctcttt 300

cgatgacctc ccattgatat ttaagtcaat aaacggtctt caatttctca agtttcagtt 360

tcatttttct tgttctatta caactttttt tacttcttgc tcattagaaa gaaagcatag 420

caatctaatc taagggcgag ctcgaattcg aactagtact gcagcacgtg accggcgcct 480

agtgttgaca attaatcatc ggcatagtat atcggcatag tataatacga ctcactatag 540

gaggaagctt ggcgctatgg catcgaagta catagtacat ccgcaaccag agcgccggca 600

cgggtcggaa actcacgaat ttaccatgcc tctcttatct cctacattga caccatccca 660

tattccatcg caaacgcctc aaattcctcc ggaagtgcca gcagaagtca gggatcgctt 720

gccccttcct gaaacgttgc ctgtggtgaa atgcatggcg agagctcgta taccgaccac 780

tcaggggccg gagatatttc tccatttgta cgagaataac gttgacaata aagagcattt 840

ggctattgtt tttggggaag atgtgcggtc gaaaacgctc tatcagaaac gtcccaatga 900

gacccagcaa gatagaatga ctcgtggtgc ttatgtgggc agattgtttc ctggaagaac 960

cgaggcagac tatgacagtg agtctaattt gagattgaat ttcgatgaaa atggccaact 1020

tatcagagat ccgagtacca cctgtagtgg tgagcccatt ttggcccgta ttcattcgga 1080

<210> 8
<211> 175
<212> PRT
<213> unknown

<400> 8

Met Ala Ser Lys Tyr Ile Val His Pro Gln Pro Glu Arg Arg His Gly
1               5                   10                  15
Ser Glu Thr His Glu Phe Thr Met Pro Leu Leu Ser Pro Thr Leu Thr
            20                  25                  30
Pro Ser His Ile Pro Ser Gln Thr Pro Gln Ile Pro Pro Glu Val Pro
        35                  40                  45
Ala Glu Val Arg Asp Arg Leu Pro Leu Pro Glu Thr Leu Pro Val Val
    50                  55                  60
Lys Cys Met Ala Arg Ala Arg Ile Pro Thr Thr Gln Gly Pro Glu Ile
65                  70                  75                  80
Phe Leu His Leu Tyr Glu Asn Asn Val Asp Asn Lys Glu His Leu Ala
                85                  90                  95
Ile Val Phe Gly Glu Asp Val Arg Ser Lys Thr Leu Tyr Gln Lys Arg
            100                 105                 110
Pro Asn Glu Thr Gln Gln Asp Arg Met Thr Arg Gly Ala Tyr Val Gly
        115                 120                 125
Arg Leu Phe Pro Gly Arg Thr Glu Ala Asp Tyr Asp Ser Glu Ser Asn
    130                 135                 140
Leu Arg Leu Asn Phe Asp Glu Asn Gly Gln Leu Ile Arg Asp Pro Ser
145                 150                 155                 160
Thr Thr Cys Ser Gly Glu Pro Ile Leu Ala Arg Ile His Ser Glu
                165                 170                 175

<210> 9
<211> 1742
<212> DNA
<213> Candida guilliermondii

<220>
<223> RIB 1 Gene

<400> 9
   caattcgagc tcggtacccg gggatccccc acacaccata gcttcaaaat gtttctactc 60
   cttttttact cttccagatt ttctcggact ccgcgcatcg ccgtaccact tcaaaacacc 120
   caagcacagc atactaaatt tccctctttc ttcctctagg gtgtcgttaa ttacccgtac 180
   taaaggtttg gaaaagaaaa aagagaccgc ctcgtttctt tttcttcgtc gaaaaaggca 240
   ataaaaattt ttatcacgtt tctttttctt gaaatttttt tttttgattt ttttctcttt 300
   cgatgacctc ccattgatat ttaagtcaat aaacggtctt caatttctca agtttcagtt 360
   tcatttttct tgttctatta caactttttt tacttcttgc tcattagaaa gaaagcatag 420
   caatctaatc taagggcgag ctcgaattcg aactagtact gcagcacgtg accggcgcct 480
   agtgttgaca attaatcatc ggcatagtat atcggcatag tataatacga ctcactatag 540
   gaggaagctt ggcgctatgg catcgaagta catagtacat ccgcaaccag agcgccggca 600
   cgggtcggaa actcacgaat ttaccatgcc tctcttatct cctacattga caccatccca 660
   tattccatcg caaacgcctc aaattcctcc ggaagtgcca gcagaagtca gggatcgctt 720
   gccccttcct gaaacgttgc ctgtggtgaa atgcatggcg agagctcgta taccgaccac 780
   tcaggggccg gagatatttc tccatttgta cgagaataac gttgacaata aagagcattt 840
   ggctattgtt tttggggaag atgtgcggtc gaaaacgctc tatcagaaac gtcccaatga 900
   gacccagcaa gatagaatga ctcgtggtgc ttatgtgggc agattgtttc ctggaagaac 960
   cgaggcagac tatgacagtg agtctaattt gagattgaat ttcgatgaaa atggccaact 1020
   tatcagagat ccgagtacca cctgtagtgg tgagcccatt ttggcccgta ttcattcgga 1080
   atgttatacg gggaaaaccg catggagtgc tcgttgcgat tgtggagaac aattcgatga 1140
   agctggtcgg ttaatgggtg aagctgggca cgggtgtatc gtgtaccttc gtcaggaagg 1200
   tcgtggaatt ggacttgggg aaaagttgaa ggcttataat ttgcaagact tgggagcgga 1260
   taccgtccag gccaatttga tgttacgaca tcctgctgat gcgagatctt tttcgctcgc 1320
   tacagccata ctcttggact tggggctcaa cgagatcaag ttgttgacca acaatcccga 1380
   taaaattgct gcagttgagg gaagaaacag agaggtcaag gtagtggaac gggtgcctat 1440
   ggtgccgttg gcatggagaa gtaagaatgg aatcaagtca aaagagatag agggctactt 1500
   gagtgctaag attgaaagga tggggcactt gcttgaaaag ccactcaaga tatgatagaa 1560
   gagatgaagt taaggactta agaaataaat gatgaattaa atgacgcaaa tgtcactact 1620
   cgattagaga aatagctata atgaagaatt ttgcatttcg caaaatttaa gataaatgca 1680
   aaaattgcaa attacgaaat atgcatatga tacaagacaa gaaaagacta ctaaaagtct 1740
   ct 1742

<210> 10
<211> 332
<212> PRT
<213> unknown

<400> 10

Met Ala Ser Lys Tyr Ile Val His Pro Gln Pro Glu Arg Arg His Gly
1               5                   10                  15
Ser Glu Thr His Glu Phe Thr Met Pro Leu Leu Ser Pro Thr Leu Thr
            20                  25                  30
Pro Ser His Ile Pro Ser Gln Thr Pro Gln Ile Pro Pro Glu Val Pro
        35                  40                  45
Ala Glu Val Arg Asp Arg Leu Pro Leu Pro Glu Thr Leu Pro Val Val
    50                  55                  60
Lys Cys Met Ala Arg Ala Arg Ile Pro Thr Thr Gln Gly Pro Glu Ile
65                  70                  75                  80
Phe Leu His Leu Tyr Glu Asn Asn Val Asp Asn Lys Glu His Leu Ala
                85                  90                  95
Ile Val Phe Gly Glu Asp Val Arg Ser Lys Thr Leu Tyr Gln Lys Arg
            100                 105                 110
Pro Asn Glu Thr Gln Gln Asp Arg Met Thr Arg Gly Ala Tyr Val Gly
        115                 120                 125
Arg Leu Phe Pro Gly Arg Thr Glu Ala Asp Tyr Asp Ser Glu Ser Asn
    130                 135                 140
Leu Arg Leu Asn Phe Asp Glu Asn Gly Gln Leu Ile Arg Asp Pro Ser
145                 150                 155                 160
Thr Thr Cys Ser Gly Glu Pro Ile Leu Ala Arg Ile His Ser Glu Cys
                165                 170                 175
Tyr Thr Gly Glu Thr Ala Trp Ser Ala Arg Cys Asp Cys Gly Glu Gln
            180                 185                 190
Phe Asp Glu Ala Gly Arg Leu Met Gly Glu Ala Gly His Gly Cys Ile
        195                 200                 205
Val Tyr Leu Arg Gln Glu Gly Arg Gly Ile Gly Leu Gly Glu Lys Leu
    210                 215                 220
Lys Ala Tyr Asn Leu Gln Asp Leu Gly Ala Asp Thr Val Gln Ala Asn
225                 230                 235                 240
Leu Met Leu Arg His Pro Ala Asp Ala Arg Ser Phe Ser Leu Ala Thr
                245                 250                 255
Ala Ile Leu Leu Asp Leu Gly Leu Asn Glu Ile Lys Leu Leu Thr Asn
            260                 265                 270
Asn Pro Asp Lys Ile Ala Ala Val Glu Gly Arg Asn Arg Glu Val Lys
        275                 280                 285
Val Val Glu Arg Val Pro Met Val Pro Leu Ala Trp Arg Ser Lys Asn
    290                 295                 300
Gly Ile Lys Ser Lys Glu Ile Glu Gly Tyr Leu Ser Ala Lys Ile Glu
305                 310                 315                 320
Arg Met Gly His Leu Leu Glu Lys Pro Leu Lys Ile
                325                 330

<210> 11
<211> 1175
<212> DNA
<213> Candida guilliermondii

<220>
<223> RIB 1 Gene

<400> 11

gggcatgcaa ttcgagctcg gtacccgggg atcccccaca caccatagct tcaaaatgtt 60

tctactcctt ttttactctt ccagattttc tcggactccg cgcatcgccg taccacttca 120

aaacacccaa gcacagcata ctaaatttcc ctctttcttc ctctagggtg tcgttaatta 180

cccgtactaa aggtttggaa aagaaaaaag agaccgcctc gtttcttttt cttcgtcgaa 240

aaaggcaata aaaattttta tcacgtttct ttttcttgaa attttttttt ttgatttttt 300

tctctttcga tgacctccca ttgatattta agtcaataaa cggtcttcaa tttctcaagt 360

ttcagtttca tttttcttgt tctattacaa ctttttttac ttcttgctca ttagaaagaa 420

agcatagcaa tctaatctaa gggcgagctc gaattcgaac tagtactgca gcacgtgacc 480

ggcgcctagt gttgacaatt aatcatcggc atagtatatc ggcatagtat aatacgactc 540

actataggag gaagcttggc gctatggcat cgaagtacat agtacatccg caaccagagc 600

gccggcacgg gtcggaaact cacgaattta ccatgcctct cttatctcct acattgacac 660

catcccatat tccatcgcaa acgcctcaaa ttcctccgga agtgccagca gaagtcaggg 720

atcgcttgcc ccttcctgaa acgttgcctg tggtgaaatg catggcgaga gctcgtatac 780

cgaccactca ggggccggag atatttctcc atttgtacga gaataacgtt gacaataaag 840

agcatttggc tattgttttt ggggaagatg tgcggtcgaa aacgctctat cagaaacgtc 900

ccaatgagac ccagcaagat agaatgactc gtggtgctta tgtgggcaga ttgtttcctg 960

gaagaaccga ggcagactat gacagtgagt ctaatttgag attgaatttc gatgaaaatg 1020

gccaacttat cagagatccg agtaccacct gtagtggtga gcccattttg gcccgtattc 1080

attcggaatg ttatacgggg gaaaccgcat ggagtgctcg ttgcgattgt ggagaacaat 1140

tcgatgaagc tggtcggtta atgggtgaag ctggg 1175

<210> 12
<211> 204
<212> PRT
<213> unknown

<400> 12

Met Ala Ser Lys Tyr Ile Val His Pro Gln Pro Glu Arg Arg His Gly
1               5                   10                  15
Ser Glu Thr His Glu Phe Thr Met Pro Leu Leu Ser Pro Thr Leu Thr
            20                  25                  30
Pro Ser His Ile Pro Ser Gln Thr Pro Gln Ile Pro Pro Glu Val Pro
        35                  40                  45
Ala Glu Val Arg Asp Arg Leu Pro Leu Pro Glu Thr Leu Pro Val Val
    50                  55                  60
Lys Cys Met Ala Arg Ala Arg Ile Pro Thr Thr Gln Gly Pro Glu Ile
65                  70                  75                  80
Phe Leu His Leu Tyr Glu Asn Asn Val Asp Asn Lys Glu His Leu Ala
                85                  90                  95
Ile Val Phe Gly Glu Asp Val Arg Ser Lys Thr Leu Tyr Gln Lys Arg
            100                 105                 110
Pro Asn Glu Thr Gln Gln Asp Arg Met Thr Arg Gly Ala Tyr Val Gly
        115                 120                 125
Arg Leu Phe Pro Gly Arg Thr Glu Ala Asp Tyr Asp Ser Glu Ser Asn
    130                 135                 140
Leu Arg Leu Asn Phe Asp Glu Asn Gly Gln Leu Ile Arg Asp Pro Ser
145                 150                 155                 160
Thr Thr Cys Ser Gly Glu Pro Ile Leu Ala Arg Ile His Ser Glu Cys
                165                 170                 175
Tyr Thr Gly Glu Thr Ala Trp Ser Ala Arg Cys Asp Cys Gly Glu Gln
            180                 185                 190
Phe Asp Glu Ala Gly Arg Leu Met Gly Glu Ala Gly
        195                 200